# EUROPEAN PATENT APPLICATION

(11) **EP 3 879 271 A1**
(43) Date of publication of application: **15.09.2021**
(21) Application number: 20162195.0
(22) Date of filing: 10.03.2020
(51) Int. Cl.: G01N 33/564, G01N 33/68

(54) **DETECTION OF KLOTHO**

(71) Applicant: SALION GmbH, 82541 Münsing (DE)
(72) Inventor: Stangl, manfred J., Dr., 82054 Sauerlach (DE); Abendroth, Dietmar, Pfofessor, 89275 Thalfingen (DE)
(74) Representative: Weickmann & Weickmann PartmbB

(57) **Abstract**

The present invention relates to an *in vitro* method for determining and/or monitoring an individual's health status by measuring the content of Klotho in saliva or tear fluid. A test kit to perform such an assay is an immunoassay test kit, especially adapted to perform a double antibody sandwich assay or a competitive assay.

## Description

The present invention relates to a diagnostic method for the determination of Klotho protein levels in an individual and to a test kit for performing such diagnostic method.

### Background of the Invention

Klotho was detected in 1997 by Makoto Kuro-o, who found that in mice, loss of this protein resulted in the early appearance of several pathological phenotypes. Mice which do not produce Klotho exhibited syndromes that resemble human ageing, including a short lifespan. Klotho has been shown to be involved in the suppression of several ageing phenotypes. A defect in Klotho gene expression in the mouse results in infertility, arteriosclerosis/vascular calcification, skin atrophy, osteoporosis, lung emphysema, acute and chronic kidney diseases, renal fibrosis, diabetes and cancer *(*Kuro-o, M., et al. (1997), Nature 390, 45-51*).* In contrast, overexpression of Klotho has been shown to increase the life-span of mice *(*Kurosu, H. et al. Science 2005, 309, p 1829*).* The Klotho protein is most highly expressed in the kidney, brain and pituitary gland, and is present in lower levels within skeletal muscle, the urinary bladder, the ovary and the testes *(*Avin, K. G., et al. (2014), Frontiers in physiology 5, 189*).*

WO2016/135295 discloses genetically modified mesenchymal stem cells expressing Klotho and contains further information with regard to the protein as well as its relevance in the context of various diseases, which information is reproduced in the following to highlight the importance of the protein for human health and well-being:
The human Klotho gene encodes a type-1 transmembrane protein of 1012 amino acids, however, by alternative splicing the gene can also be expressed in a secreted form. Thus, Klotho exists is two forms: membrane Klotho and secreted Klotho. Membrane Klotho functions as a receptor for a hormone that regulates excretion of phosphate and synthesis of active vitamin D in the kidney. Secreted Klotho on the other hand is a humoral factor with pleiotropic activities, including suppression of growth factor signaling, suppression of oxidative stress, and regulation of ion channels and transporters.

Fibroblast growth factor 23 (FGF23), a member of the fibroblast growth factor (FGF) family, was identified to be elevated in patients with autosomal dominant hypophosphatemic rickets (ADHR). FGF23 functions as a phosphaturic hormone and a counter-regulatory hormone for vitamin D (calcitriol) in a Klotho-dependent manner. Hyperphosphatemia leads to stenosis of blood vessels, myocardial infarction, stroke and a major shortening in life expectancy in patients with chronic kidney disease (CKD). The absence of FGF-signaling in the kidney results in increased serum levels of phosphate. It has been found that each of the secreted and the membrane-bound form of Klotho forms a complex together with the FGF-receptor and thereby increases FGF23-dependent signaling *(*Kurosu et al., Journal of Biological 2006, 281(10): 6120-61*).* Klotho's function as a cofactor for FGF23 signaling is important for the regulation of the phosphate serum levels.

Furthermore, Klotho functions through the modulation of various signaling pathways including that of insulin growth factor 1 (IGF-1). One effect of Klotho is the increase in cellular resistance to oxidative stress which is involved in many different pathological processes. It has been shown that through the modulation of the cellular response to oxidative stress Klotho also acts protectively in the context of neurodegenerative diseases such as Alzheimer's Disease *(*Zeldich, E. et al., J Biol Chem 2014, 289(35):24700*)* and diabetes mellitus. Klotho also has been suggested to be a repressor of collagen synthesis and therefore might be beneficial in the context of fibrosis *(*Ghosh, A. K. et al. Exp Biol Med 2013, 238(5):461*).*

It has been shown that Klotho expression is silenced in several kinds of cancer cells, which is associated with enhanced cell growth and the formation of cancer metastasis *(*Camili et al. Pigment Cell & Melanoma Res 2011, 24(1), p 75*;* Wang et al; Am J Cancer Res 2011, 1(1):111*,* Lee et al, Molecular Cancer 2010, 9:109*).* In contrast, overexpression of Klotho in cancer cells can inhibit cell growth and can promote apoptosis of cancer cells *(*Chen. B. et al, J of Exp and Clin Cancer Res 2010, 29:99*).* Furthermore, Klotho up-regulation indirectly stimulated by administration of renin-angiotensin system inhibitors or other compounds can lead to suppression of renal fibrosis *(*Ming Chang Hu et al, Contrib Nephrol 2013, 180:47*)* and there appears to be an inherent low Klotho expression in diabetic rat models *(*Meng Fu Cheng et al, Journal of Biomedicine and Biotechnology. 2010, 513853*).*

Further research relating to various specific diseases in which alterations in Klotho levels have been observed is summarized in the following:

### Background on Chronic Kidney Disease (CKD):

CKD is a growing international health problem, affecting e.g. more than 26 million Americans. In patients with CKD renal Klotho RNA is decreased. This clinical observation was confirmed in numerous preclinical models, showing that unilateral nephrectomy and contralateral ischemia reperfusion injury downregulates renal klotho protein and mRNA expression. The same reduction in klotho expression was shown in a chronic glomerulonephritis model. Klotho overexpression improved renal function and ameliorated renal histology in this model *(*Hu, M. C, et al. (2011), Journal of the American Society of Nephrology: 22,124-136*,* Haruna, Y., et al. (2007) PNAS, 104, 2331-2336*).*

In CKD patients, there is a very high prevalence of coronary artery calcification, which increases cardiovascular morbidity and mortality. The Klotho-FGF 23 axis plays an important role in vascular mineralization *(*Stompor. T. (2014) World journal of cardiology 6, 115-129*).* Increased cardiovascular (CV) morbidity and mortality is well documented in chronic kidney disease (CKD). In a survey among 1,120,295 adults in the San Francisco Bay Area, a strong correlation between renal function (estimated glomerular filtration rate, GFR) and cardiovascular events was found *(*Go, A. S., et al. New England Journal of Medicine, 2004; 351: 1296-1305*).*

In the Renal Research Institute (RRI)-CKD Study of adults with moderate to severe CKD (Stages 3-5) enrolled between June 2000 and February 2006 (n=834) the authors found that heart rate variability is predictive for clinical outcome and cardiovascular disease (CVD) *(*Chandra, R, et al. (2012) official publication of the European Dialysis and Transplant Association-European Renal Association 27, 700-709*).* Chronic kidney disease (CKD) is therefore a major risk factor for cardiovascular disease leading to increased morbidity and shortening of lifespan. Klotho expression is markedly reduced in kidneys from patients suffering from CKD. Restoring Klotho expression by infusion of MSC-Klotho (mesenchymal stem cell derived Klotho) may improve kidney function and thereby reduce the risk for cardiovascular death.

### Background on Cardiovascular Disease:

Cardiovascular disease (CVD) is a prevalent condition in general population and the first cause of death overall. Klotho has been proposed as a key regulator of the development of CVD. In the few clinical studies made, it has been observed a relationship between low levels of soluble Klotho and the occurrence and severity of CVD, as well as a reduction of cardiovascular risk when they are high. Also, different polymorphisms of human Klotho gene have been related to the incidence of cardiovascular events. Moreover, several experimental studies indicate that this protein acts in the maintenance of vascular homeostasis *(*Yamamoto M. et al., J Biol Chem. 2005; 280: 38029-38034). Klotho improves endothelial dysfunction through promotion of NO production and mediates anti-inflammatory and anti-aging effects such as suppression of adhesion molecules expression, attenuation of nuclear factor-kappa B or inhibition of Wnt signaling. Klotho regulates expression levels of the endothelial NO synthase (eNOS). Six et al. recently observed that attenuation mediated by Klotho of FGF23 or phosphate-induced vasoconstriction is abolished by adding nitro-L- arginine, a competitive inhibitor of NOS. Moreover, they observed that exposure of HUVECs to Klotho increased NO production and induced eNOS phosphorylation and iNOS expression. Interestingly, Klotho was able to increase H202 production in cultured human VSMCs, which suggests a more complex effect of this protein on the regulation of vascular tone through mediation of a ROS/NO balance *(*Six I et al. (2014), PLoS One. 2014; 9:e93423*).*

Furthermore, this protein is related to the attenuation of vascular calcification as well as prevention of cardiac hypertrophy. The expression of this protein in the vascular wall implies a new scenario for the treatment of vascular disorders. Klotho protein is therefore related to CVD and plays a role in the maintenance of functional vascular integrity *(*Martin-Nunez. M. (2014) World J Cardiol. 6(12): 1262-1269*).*

### Background on Morbus Alzheimer (AD):

Neurodegenerative diseases, especially Morbus Alzheimer (AD), are increasing in the western world. The Alzheimer's association estimates that in the USA Alzheimer's is the 6th leading cause of death, that every 67 seconds someone is diagnosed with Alzheimer's and that the cost for medical treatment and caregiving for these patients will exceed 1.1 trillion US $ by 2050. AD is characterized through the loss of neurons and synapses, but also through the generation of neurotoxic amyloid beta peptides (Aβ-plaques) and their deposition along with neurofibrillary tangle formation. There is growing evidence, that the deposition of amyloid is the central hallmark of the disease. Activated astrocytes start an inflammatory reaction by producing proinflammatory cytokines like IL-6, IL-I and TNF-a. All this starts with an improper reaction to oxidative stress, accumulation of oxygen free radicals, hyperglycemia and insulin resistance *(*Kosales-C'orral, S., el al. (2015) Oxidative medicine and cellular longevity, 985845*).*

Recently it was shown, that in the cerebrospinal fluid of AD patients, the concentration of the anti-aging protein klotho is significantly lower than in younger patients or in old patients without AD *(*Semba, R. D., et al. (2014) Neuroscience letters 558, 37-40*).*

In the brain, Klotho protein is localized at the choroid plexus, where the protein is dominantly localized at the apical plasma membrane of ependymal cells. In kl-/- mouse brain, reduction of synapses was evident in the hippocampus, suggesting a role of Klotho as a humoral factor in the cerebrospinal fluid. Klotho protein in the kidney is localized at the distal renal tubules *(*Li S A. et al. (2004) Cell Structure and Function 29, 91-99*).*

Chen et al demonstrated that loss of Klotho expression leads to cognitive deficits. They found significant effects of Klotho on oligodendrocyte functions, including induced maturation of rat primary oligodendrocytic progenitor cells (OPCs) in vitro and myelination. Klotho increased OPC maturation. In vivo studies of Klotho knock-out mice and control littermates revealed that knock-out mice have a significant reduction in major myelin protein and gene expression. By immunohistochemistry, the number of total and mature oligodendrocytes was significantly lower in Klotho knock-out mice. At the ultrastructural level, Klotho knock-out mice exhibited significantly impaired myelination of the optic nerve and corpus callosum *(*Chen, C. D., et al. (2013) The Journal of neuroscience 33, 1927-1939*).*

### Background on Multiple Sclerosis (MS):

MS is a complex disease of the CNS that is characterized by heterogeneous pathologies composed of both inflammatory and neurodegenerative components. The most common histopathological feature at early stages of the disease includes intermittent episodes of acute inflammation within patches of white matter, resulting in demyelination. Myelin is critical for maintaining efficient axonal conduction and oligodendrocytes, the myelin producer and maintainer of axonal health within the CNS, are damaged or destroyed in MS patients. Endogenous oligodendrocyte precursor cells (OPCs) are found to be universally dispersed within the human CNS and can be found in high density within some subacute lesions during early stages of MS.

Progressive MS is the latest stage of the disease, characterized by a gradual worsening of symptoms without remission. Severe neurological impairments dramatically reduce the quality of life for the individual, and this is mainly attributed to expanding cortical lesions impacting motor function. Pathologically, there is widespread axonal degeneration and grey matter neuropathy. Diffuse white and gray matter inflammation has been reported, correlating, in part, to global microglial activation as well as the presence of T cells. B cells and myelin-laden macrophages. Furthermore, there is an overall failure of OPCs to efficiently remyelinate damaged white and gray matter areas, dramatically reducing the possibility for recovery *(*Chang, A., et al. (2002) The New England journal of medicine 346. 165-173*).* Klotho enhances the maturation of OPCs into mature oligodendrocytes *(*Chen, C. D., et al. (2013) The Journal of neuroscience 33, 1927-1939*).*

### Background on Parkinson Disease (PD):

PD is a progressive neurodegenerative disorder clinically characterized by the cardinal symptoms of resting tremor, bradykinesia, cogwheel rigidity, and postural instability. Responsiveness to L-3,4-dihydroxyphenylalanine (L-DOPA) and brain imaging distinguish PD from other disorders. The pathological hallmarks of PD are loss of dopaminergic cells in the substantia nigra, pars compacta and subsequent loss of dopamine innervation in the striatum. Motor symptoms are the most obvious consequence of this nigrostriatal neurodegeneration. However, not only the basal ganglia but also other parts of the central nervous system as well as the autonomic nervous system are affected. A wide range of resulting non-motor symptoms can affect the patient's quality of life. There is also a broad consensus that neurodegenerative processes in PD start many years before the actual onset of clinical symptoms. The phenotypical over-lapping between familial and idiopathic PD is sufficient to dissect the commonly involved pathways. These include mitochondrial dysfunction, oxidative stress, protein misfolding, protein degradation, protein aggregation, and inflammation.

Kosakai et al. demonstrated that the number of tyrosine-hydroxylase-positive dopaminergic neurons in the substancia nigra, pars compacta, the ventral segmental area and the striatal dopamine level in klotho-insufficient mice, were significantly decreased in age-dependent fashion. These phenotypic features were completely rescued by vitamin D restriction, indicating that abnormal increase in active vitamin D biosynthesis by Klotho insufficiency induces degeneration of dopaminergic neurons *(*Kosakai, A., et al. (2011) Brain research 1382, 109-117*).*

Due to Klotho's contribution to various signaling pathways and its important functions in many organ systems and based on the observation of reduced levels of the protein in the context of various diseases, novel treatments including Klotho administration may represent promising therapeutic approaches. E.g., WO2016/135295 provides mesenchymal stem cells which can be administered to a patient to enable treatment of the above described diseases, but also other conditions in individuals for which the administration of Klotho is beneficial.

In addition to a possible treatment of diseases, in which the Klotho level is known to be decreased, the protein itself has also been considered as some kind of marker for health and well-being of an organism. While it is a usual concept in the diagnostic field to determine biological markers for specific diseases, a monitoring of molecules which can indicate a good health or at least a well-being with regard to various major health aspects has been neglected. Fantuzzi (frontiers in IMMUNOLOGY, July 2014, Volume 5, Article 351) focusses on the "Sound of health" or, actually, the silence of health which up to now has not sufficiently found resonance. He reports that, rather, messages of distress have been favored over molecules which can be considered as markers for a well-balanced metabolism and lack of distress in an organism. Fantuzzi mentions Klotho as one of such candidate messengers of health as it has been observed that in a state of health and a lack of distress this molecule is optimally produced, released and bioactive, whereas any sort of stress leads to reduced levels of Klotho. An optimal Klotho level in a person can be a significant indication for good health, e.g. when determined in a regular health screening.

While, especially for more severe conditions, administration of Klotho-producing mesenchymal stem cells provides for protein expression and for arriving at protein levels which can be considered curative, in certain conditions prophylactic and also therapeutic increase of the Klotho levels in individuals can also be achieved by physical training. E.g. Matsubara et al. (AM J Physiol Heart Circ Physiol. 306:H348-H355, 2014*)* report the increase of plasma Klotho levels and reduced arterial stiffness in postmenopausal women after aerobic exercise training. Also Ji et al. (Experimental and Therapeutic Medicine 16:3511-3517, 2018*)* predict positive effects on aging and aging-related diseases due to aerobic exercise and the concomitant promotion of Klotho expression. Furthermore, Tan et al. (Journal of Circulating Biomarkers, Volume 7:1-7,2018*)* observed increased Klotho serum levels in healthy volunteers after high intensity physical exercise training. Even a single training session was found to induce formation of Klotho. Finally, Avin et al. (frontiers in Physiology, Volume 5, Article 189, June 2014*)* summarize earlier published effects and demonstrate impressively the effect of exercise, especially of skeletal muscle, on Klotho expression and resulting increased Klotho levels in plasma.

These observations link the life-prolonging effect of physical activity, especially in patients with certain diseases like diabetes, to the presence and plasma levels of Klotho. Santos-Dias et al. (Br J Sports Med 2016; 0:1-2) show that even a single exercise session significantly induces increased serum Klotho levels in men and women. Mostafidi et al. (Nephro Urol Mon., 2016 Jan; 8(1): e30245) report a significantly higher concentration of free Klotho in plasma for a group of athletes compared to a group of non-athletes, while other elements of serum did not significantly differ between the two groups.

The observation that aerobic exercise and especially muscle strength training can increase the serum Klotho level is a very promising finding. These results indicate that certain conditions, but also the general health can be vastly improved by physical training and body workouts and further results also indicate that, especially in older adults or also in persons suffering from neurodegenerative diseases, raising the plasma Klotho levels can improve the cognitive situation or at least slow down cognitive decline. (Shardell et al., J Gerontolog A Biol Sci Med Sci, 2015, 1-6*;* Cheng et al., Acta Neurobiol Exp 2015, 75: 60-71*).*

There is no doubt that the Klotho level is an important indicator of health or disease and can help in achieving longevity in individuals by raising too low levels to optimal values. A drawback of the current situation is, however, that measuring Klotho levels in an individual needs to be performed on blood samples, which are usually drawn in a medical practice and analysed in a clinical laboratory. Determining the Klotho level is important not only for an initial diagnosis but needs to be monitored during or after treatment of a diseases with commonly applied medication or by administration of Klotho, or during efforts to improve a person's health by physical exercise. Especially for a regular observation of the Klotho levels in individuals, the current situation is not satisfactory and it was an object of the invention to provide improved means to determine the Klotho levels in a person in a cost-effective and easily applicable manner, which at best can even be performed during or directly after a physical exercise to monitor the Klotho level.

### Summary of the Invention

In a first aspect, the invention relates to a method for determining and/or monitoring an individual's health status by measuring the content of Klotho in a body fluid, wherein the body fluid is selected from saliva and tear fluid.

In a second aspect, the invention relates to a test kit, which is characterized by containing the required reagents for performing an immunoassay.

### Detailed description of the invention

The present invention solves the above-mentioned object by providing an improved method for the determination of Klotho levels in a patient, and it especially provides methods and means in the form of test kits for performing the diagnostic method. The tests can easily be performed by the individuals or patients on their own and at any given time at which such determination seems appropriate.

The inventive method is based on the surprising finding of the inventors that the Klotho level in especially saliva proportionally corresponds very well with the Klotho levels that have been determined in serum samples. Accordingly, the method of the present invention is preferably performed with saliva.

The object to provide a test which is painless, inexpensive, more reliable, easier and safer than approaches based on serum or urine with an impact of molecular diagnostic is met by the method of the present invention. The present method was compared with the HPLC-technique whereby comparable results were found. The already existing results are surprising in that there is a significant difference of Klotho levels in serum and saliva between patients and healthy volunteers.

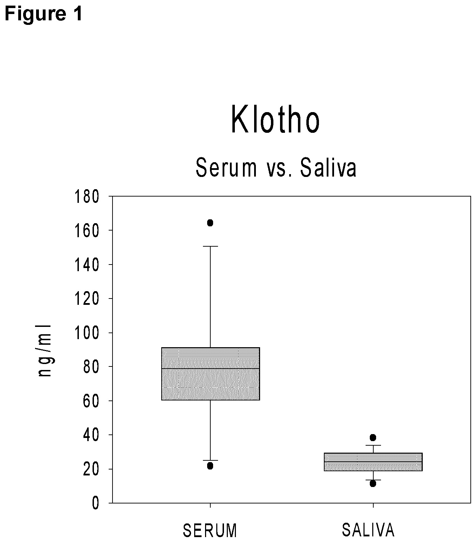

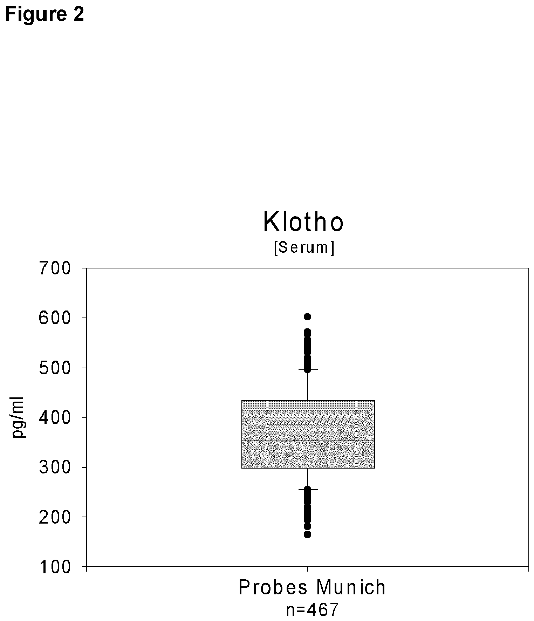

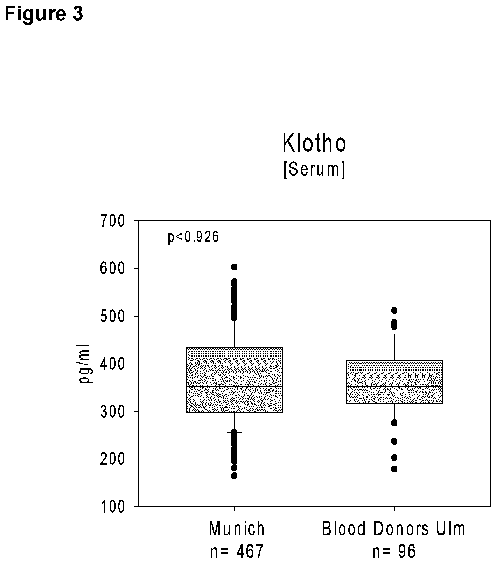

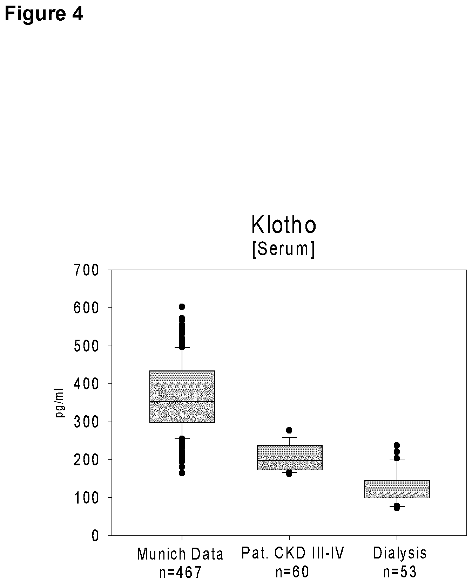

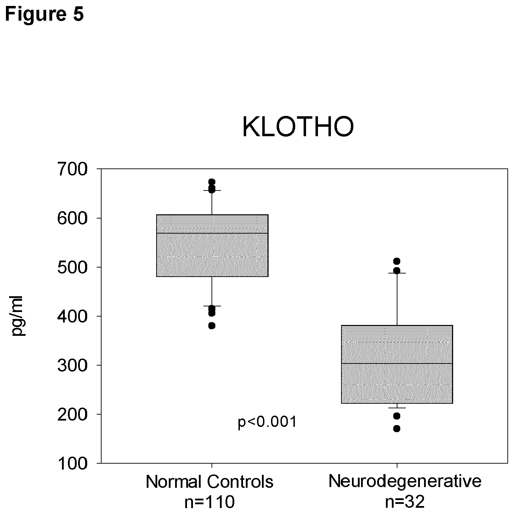

While the actual values for serum and saliva differ to some extent in the same individual and in the same group of persons, a reliable relation between Klotho levels in serum and other body fluids, especially saliva, could be determined (see Figs. 1 to 5). This observation proves that the Klotho protein is present at a significant and meaningful level in in saliva, which is not the case for many other proteins (Tékus et al., Acta Biol Hung, 2012; 63 (Suppl1): 89-98*;* Ellis et al., N Z Med JK, 2012; 125(1353): 47-58*;* Volkery et al., Vet Rec, 2012; 171(8): 195*;* Holten-Anderson et al., Scand J Gastroenterol, 2012; 47(10): 1234-41*;* Rahnama et al., Endokr Res, 2012 Aug 15 epub*;* Pant Pai et al., Lancet Infect Dis, 2012; 12(5): 373-80*;* Mahboobi et al., J Oral Pathol Med, 2012; 41: 505-16*;* Yen Bee Ng et al., FEMS Immunol Med Microbiol, 2007; 49(2): 252-60*;* Castagnola et al., Acta Otorinologica Italica, 2011; 31: 347-57*):*

Taking advantage of this observation, the present invention for the first time provides a reliable test method and a test kit for monitoring an individual's health status. In certain embodiments of the present invention, this health status is considered to include an existing or a previous disease or condition which correlates with a low or decreased Klotho level, or a disposition for such disease or condition. Such diseases or conditions which are accompanied by a comparatively low Klotho level have been described in the prior art and in some detail in the introductory part of the present specification. This does, however, not exclude further diseases for which a decreased Klotho level might only be recognized in the future. Accordingly, the detection of a comparatively low Klotho level by the inventive method might also be an indication that a person's health is not satisfactory even though specific disease symptoms have not yet been observed. Thus, further diagnostic methods can be applied to detect or exclude severe diseases.

In preferred embodiments, the disease or condition, which is accompanied by or causes decreased Klotho levels compared to healthy persons, is selected from at least one of cancer, inflammatory diseases, chronic kidney disease (CKD), neurodegenerative diseases, chronic heart disease, organic fibrosis, atherosclerosis, dementia, diabetes mellitus, erectile dysfunction, autoimmune diseases or autoimmune-related diseases, sepsis and also premature aging and other age-related diseases.

Neurodegenerative diseases as well as preconditions for neurodegenerative diseases for which a low Klotho level determined by the method of the present invention can be an indication include Morbus Alzheimer, Parkinson disease, amyothrophic lateral sclerosis (ALS) and Huntington disease. In further preferred embodiments, the health status of a person is affected by an autoimmune disease or an autoimmune-related disease including type 1 diabetes, rheumatoid arthritis (RA), multiple sclerosis (MS), systemic lupus erythematosus, auto-immune encephalomyelitis, auto-immune nephritis, such as lupus nephritis and IgA nephropathy, osteoarthritis or other diseases which activate the inflammasome or constituents thereof in a patient.
The method of the present invention can be applied to either detect the risk of the presence of such diseases or to monitor the progress of the disease and/or the effect of a medical treatment.

Especially with regard to cases, in which the inventive method is used in the context of an existing or previous disease, it is preferred to monitor and determine the Klotho level before, during and/or after the treatment. In this context, a treatment includes administration of Klotho as well as administration of other therapeutic agents or a combination thereof. As mentioned above, Klotho can be either applied as a protein or the formation of Klotho can be promoted by e.g. administration of stem cells which express Klotho. If the Klotho level can be raised during treatment, the health status of the person is clearly improved.

Finding of a low level of Klotho in an otherwise healthy person can also be considered a strong indication that such person is not sufficiently physically active. Such lack of physical exercise might lead to more or less severe health problems and premature aging, if this person does not change his or her lifestyle. Accordingly, a further preferred embodiment of the present invention is the in vitro method in which the Klotho content is determined to monitor the effect of physical activity on the health status of an individual. As explained above, physical activity and especially physical training, muscle training and endurance training have been shown to increase the Klotho level which reflects also an increase in the health status and especially the cognitive abilities of an individual.

The inventive in vitro method can also be used to determine the biological age of an individual. Again, determining such biological age can help to address possible health issues and unhealthy behavior and, thus, to improve longevity by then taking the required actions which results in increasing and adjusting the Klotho level.

The actual correlation between the values obtained in an immunoassay using serum vs. saliva can be easily determined or verified for any assay format. For instance, a relation between Klotho in serum and saliva has been determined to be 3.6:1 when measured by the TRF method (time related fluorescence immunoassay) (Fig. 1). Thus, a calibration of the test method provides a relation of "normal" values in healthy persons in e.g. saliva compared to serum.

"Normal" values can also be determined by providing test results for an appropriately similar cohort of persons, which are considered healthy or the Klotho level of which ranges within a certain acceptable deviation from a mean value of a cohort. Cohorts can be selected from persons of a similar age, the same sex, similar living circumstances etc. Furthermore, values reported in the literature can be included to select "normal" values under certain circumstances.

The in vitro method of the present invention will preferably be accompanied by at least one therapeutic measure if the detected Klotho level is below a certain threshold value, which can be derived e.g. as stated above. The therapeutic measure includes administering Klotho to an individual, or otherwise increasing the Klotho level in such individual for which a Klotho level has been determined, which based on the proper relation as explained above corresponds to a serum value of below 100 pg/ml. Preferably therapeutic measures to raise the Klotho level should already be initiated if the Klotho level corresponds to a serum level below 200 pg/ml and most preferably at a level of less than 300 pg/ml The Klotho levels determined in saliva for which a therapeutic measure has to be considered as desirable are thus reduced by a factor of 3.6.

Preferably, administering of Klotho in the context of the present invention includes administering a therapeutically effective number of mesenchymal stem cells, which produce Klotho, or a therapeutically effective number of cells containing a nucleic acid vector, which comprises a region encoding a Klotho protein, said region being operably linked to a promotor or promotor/enhancer combination, wherein said vector containing cells express Klotho. Also, Klotho-producing viral vectors or inducing Klotho expression via CRISPR-Cas9 gene editing can be considered in the context of the present invention in order to elevate Klotho levels in an individual. In other embodiments, Klotho protein is delivered or administered directly to a patient, wherein the Klotho protein preferably is recombinantly produced by biotechnological methods.

The amount of Klotho to be administered depends on the actual level determined in an individual or a patient and is preferably suitable to raise the level to more than 340pg/ml when determined in serum and, correspondingly, to more than 85 pg/ml, preferably more than 100 pg/ml when determined in saliva.

In another preferred embodiment, instead of an administration of the Klotho protein to the patient or inducing expression thereof in the patient by biotechnological methods as outlined above, the present invention also includes subjecting the individual to increased physical activity. Especially a physical training and especially a muscle training is indicated in such patients if the determined Klotho level is below a value corresponding to "normal" levels determined in healthy groups of persons and especially values as outlined above..

According to the present invention, the Klotho level is preferably determined via an immunoassay including at least one antibody or aptamer, which specifically binds Klotho. The inventive method preferably includes determination of Klotho levels by a double antibody sandwich assay or a competitive assay. Both assays are most preferably performed as a lateral flow assay.

The specific design of the assay is not decisive and appropriate test formats are available to the skilled person. Some specific preferred embodiments are described in more detail below, as a further subject of the present invention is a test kit for performing the inventive method. Such test kit contains the required reactants for performing an immunoassay.

In principle, any immunoassay format can be applied to perform the inventive method. In preferred embodiments, the test is performed as a double antibody sandwich assay or a competitive assay. Assay environments including solid phases, antibodies but also labels for use in such methods are also well known to the skilled person and there is no restriction to the applicable test formats. However, in a very preferred embodiment of the present invention, the test format provided by the test kit of this invention, is suitable for a lateral flow assay format. This type of test and corresponding test kits are described in more detail as follows:

### Lateral Flow Assay

As mentioned, the kits for performing the *in vitro* method as disclosed herein may be based on different principles. One of the preferred principles is known as Lateral Flow Immunochromatographic Assay. Such a Lateral Flow Immunochromatographic Assay can be easily performed by the patient/sportsman without the help of a doctor or other medically trained person.

Lateral flow tests are simple devices intended to detect the presence (or absence) of a target analyte sample without the need for specialized and costly equipment, though many lab based applications exist that are supported by a reading equipment. Typically, these tests are used for medical diagnostics either for home testing, point of care testing (POC), or laboratory use. A widely spread and well known application is the home pregnancy test.

The technology is based on a series of capillary beds, such as pieces of porous paper or sintered polymer. Each of these elements has the capacity to transport fluid (e.g., saliva) spontaneously, the first element (the sample pad) acts as a sponge and holds an excess of sample fluid. Once soaked, the fluid migrates to the second element (conjugate pad) in which the manufacturer has stored the so called conjugate, preferably a dried format of bio-active particles (see below), preferably in a salt-sugar matrix, that contains everything to guarantee an optimized chemical reaction between the target molecule (e.g., Klotho) and its chemical partner (e.g. antibody) that has been immobilized on the particle's surface. While the sample fluid dissolves the salt-sugar matrix, it also dissolves the particles and in one combined transport action the sample and conjugate mix, while flowing through the porous structure. In this way, the analyte binds to the particles while migrating further through the third capillary bed. This material has one or more areas (reaction or capture zones, often called stripes) where a third molecule has been immobilized by the manufacturer. By the time the sample-conjugate mix reaches these strips, analyte has been bound on the particle and the third 'capture' molecule binds the complex. After a while, when more and more fluid has passed the stripes, particles accumulate and the stripe-area changes color. Typically there are at least two stripes: one (the control zone) that captures any particle and thereby shows that reaction conditions and technology worked fine, the second contains a specific capture molecule and only captures those particles onto which an analyte molecule has been immobilized. After passing these reaction zones the fluid enters the final porous material, the wick (or waste reservoir) that simply acts as a waste container.

Such test design can be adjusted to the nature of the immunoassay which is performed using the test kit. Lateral Flow Tests can operate as either competitive or sandwich assays.

In principle, any colored particle can be used for the conjugate, however, latex (blue color) or nanometer sized particles of gold (red/black color) are most commonly used. The gold particles are red in color due to localized surface plasmon resonance. Fluorescent or magnetic labeled particles can also be used, however these require the use of an electronic reader to assess the test result.

The sample first encounters colored particles in the conjugate or reagent pad which are labeled with antibodies raised to the target analyte. The test line in the capture zone will also contain antibodies to the same target, although it may bind to a different epitope on the analyte. The test line will show as a colored band in positive samples. An example of the sandwich assay is the sandwich ELISA. While not strictly necessary, most test kits preferably incorporate a second line which contains an antibody that picks up free particles like latex/gold in order to confirm the test has operated correctly.

In a preferred embodiment the single components of the lateral flow assay are adapted in such a manner that the presence of klotho is indicated only when more than a certain threshold value of klotho is present in the sample.

A preferred test kit of the present invention consists of the following components:
1. As sample pad an absorbent pad onto the test sample (saliva, tear fluid, urine) is applied
2. Conjugate or reagent pad - this contains antibodies specific to the target analyte Klotho and is conjugated to labelled, preferably colored, particles (usually colloidal gold particles, or latex microspheres)
3. Reaction or capture zone: a membrane - typically a hydrophobic nitrocellulose or cellulose acetate membrane onto which anti-target analyte antibodies are immobilized in a line across the membrane as a capture zone or test line (a control zone may also be present, containing antibodies specific for the conjugate antibodies)
4. Wick or waste zone or reservoir - a further absorbent pad designed to draw the sample across the reaction membrane by capillary action and collect it.

The components of the strip are usually fixed to an inert backing material and may be presented in a simple dipstick format or within a plastic casing with a sample port and reaction window" showing the capture and control zones.

There are two preferred embodiments of the test kits (lateral flow immunoassay) used in the method of the present invention:

### a. Double Antibody Sandwich Assays

In this formal the sample migrates from the sample pad through the conjugate pad where any Klotho as the target analyte present will bind to the conjugate. The sample then continues to migrate across the membrane until it reaches the capture zone where the target/conjugate complex will bind to the immobilized antibodies producing a visible line on the membrane, the sample then migrates further along the strip until it reaches the control zone, where excess conjugate will bind and produce a second visible line on the membrane. This control line indicates that the sample has migrated across the membrane as intended. Two clear lines on the membrane show a positive result. A single line in the control zone is a negative result. Double antibody sandwich assays are most suitable for larger analytes such as bacterial pathogens and viruses, with multiple antigenic sites. For the present invention a suitable pair of antibodies is selected which bind to different epitopes on Klotho.

Such antibodies can be chosen amongst the commercially available products or produced by raising antibodies, especially monoclonal antibodies, against different Klotho epitopes by methods known in the art. When the test methods or kits suitable for performing such method use antibodies which bind specifically to Klotho, the term "antibody" means not only antibodies artificially produced for example by immunization of a laboratory animal like rabbit, sheep or goat. It comprises also in a preferred embodiment monoclonal antibodies produced according to the hybridoma technology. Moreover, the term "antibody" comprises also antigen-binding fragments of antibodies such as recombinant produced antigen-binding fragments. Such constructs can e.g. be produced by phage display and technologies derived therefrom.

### b. Competitive Assays

Competitive assays are primarily used for testing small molecules and differ from the double antibody sandwich format in that the conjugate pad contains antibodies that are already bound to the target analyte, or to an analogue of it. If the target analyte is present in the sample it will therefore not bind with the conjugate and will remain unlabeled. As the sample migrates along the membrane and reaches the capture zone an excess of unlabeled analyte will bind to the immobilized antibodies and block the capture of the conjugate, so that no visible line is produced. The unbound conjugate will then bind to the antibodies in the control zone producing a visible control line. A single control line on the membrane is a positive result. Two visible lines in the capture and control zones is a negative result. However, if an excess of unlabeled target analyte is not present, a weak line may be produced in the capture zone, indicating an inconclusive result. Competitive assays are most suitable for testing for small molecules, such as mycotoxins, unable to bind to more than one antibody simultaneously.

Taking into account these different test formats which can be used for a method according to the present invention, the test kit is characterized by containing a solid phase comprising:
a) a sample pad for application of a sample to be tested,
b) a conjugate or reagent pad comprising a conjugate of a Klotho specific antibody, which conjugate comprises a detectable label, or a conjugate of such antibody/label conjugate and Klotho,
c) a reaction or capture zone on which an antibody specific to the Klotho protein is immobilized, and
d) a wick or waste reservoir for collecting sample fluid and sample components and surplus reagent or conjugate,
   and optionally further,
e) a control zone comprising an antibody specifically binding to the conjugate of step b), however, not to Klotho.

There are a number of variations on lateral flow technology. The capture zone on the membrane may contain immobilized antigens or enzymes - depending on the target analyte - rather than antibodies. It is also possible to apply multiple capture zones to create a multiplex test.

Within the context of the invention, the term antibody is meant to also comprise Klotho-binding antibody fragments or also aptamers. Thus, the embodiments as outlined above can in alternative preferred embodiments also encompass such antibody fragments or aptamers in the zones b), c) and e) as described above.

Lateral flow immunoassays are simple to use by untrained operators and generally produce a result within 15 minutes. They are very stable and robust, have a long shelf life and do usually not require refrigeration. They are also relatively inexpensive to produce. These features make them ideal for use at the point-of-care and for testing samples in the field, as well as in the laboratory. However, their sensitivity is limited without additional concentration or culture procedures. There are quantitative tests available, but the object of the present invention preferably is a qualitative test for saliva or tear fluid within a certain range. In this context, the preferred test kit is adjusted to measure Klotho only if present above a certain concentration. Below such concentration the test kit will show a negative result.

### Description of the Figures

**Figure 1** shows the relation between Klotho in serum and saliva measured by the TRF method (time resolved fluorescence immunoassay) to be about 3.6 to 1.
**Figure 2** shows the results of the determination of Klotho levels in the serum of 467 probes obtained from healthy persons in Munich (n = 467, min 364, 39 pg/ml, range 164-601 measured by Cloud Clone ELISA).
**Figure 3** shows a comparison of the data obtained in Munich for 467 probes compared to a cohort of blood donors in Ulm including 96 probands. The Klotho serum level was detected by the Cloud Clone ELISA. There might be a difference between the groups concerning age and gender in which the younger the cohort the higher the Klotho values. There is also a small difference between female and male probands in favour of higher levels for the males.
**Figure 4** shows a comparison between the Munich data and Klotho serum levels of patients with chronic kidney disease stage III and IV and patients at dialysis. In patients, the inventors as well as others (e.g. Wang et al., BioMed Research International, Vol 2018, Article ID9481475*;* Rotondi et al., International Journal of Endocrinology, Vol 2015, Article ID872193*;* and Pedersen et al.; Clinical Biochemistry, 46 (2013) 1079-1083*)* found a strong correlation between renal function and Klotho concentrations in the peripheral blood as shown in the following table:

**Table 1: e-GFR in relation to Klotho concentrations according to the international CKD-grade definitions**

| CKD | e-GFR | Mean [pg/ml] | Range |
|---|---|---|---|
| 1-2 | > 90 | 398 | 385 - 410 |
| 3 | 30-59 | 349 | 332 - 364 |
| 4 | < 30 | 338 | 291 - 361 |
| 5 | < 15 | | |

**Figure 5** shows the Klotho levels in serum measured in normal controls and in patients with neurodegenerative disease.

For the results presented in the above figures, the Klotho levels were determined using Cloud Clone Corporation's enzyme-linked immunosorbent assay SEH757HU. All reagents and materials, reagent and sample preparation and assay steps as well as the calculation were according to the instruction manual supplied with the test kit.

## Claims

1. In vitro method for determining and/or monitoring an individual's health status by measuring the content of Klotho in saliva or tear fluid.

2. In vitro method according to claim 1, in which the individual's health status includes an existing or previous disease or condition which correlates with a low or decreased Klotho level or a disposition for such disease or condition.

3. In vitro method according to claim 2, wherein the disease or condition is selected from at least one of cancer, inflammatory diseases, chronic kidney disease (CKD), a neurodegenerative disease, chronic heart disease, organ fibrosis, arteriosclerosis, dementia, diabetes mellitus, erectile dysfunction, an autoimmune disease or an autoimmune-related disease, sepsis, premature aging and other age related diseases.

4. In vitro method according to claim 3, wherein the neurodegenerative disease includes Morbus Alzheimer, Parkinson disease, amyothrophic lateral sclerosis (ALS) and Huntington's disease.

5. In vitro method according to claim 3, wherein the autoimmune disease or autoimmune-related disease includes type 1 diabetes, rheumatoid arthritis (RA), multiple sclerosis (MS), systemic lupus erythematosus, auto-immune encephalomyelitis, auto-immune nephritis, such as lupus nephritis and IgA nephropathy, osteoarthritis or other diseases which activate the inflammasome in a patient or constituents thereof.

6. In vitro method according to anyone of claims 1 to 5, wherein the determining and/or monitoring of an individual's health status is performed in the context of a therapeutic treatment of a disease by measuring the content of Klotho before, during and/or after the treatment.

7. In vitro method according to claim 1, in which the Klotho content is determined to monitor the effect of physical activity, especially physical training, muscle training and/or endurance training on the health status of an individual.

8. In vitro method according to claim 1, for determining the biological age of an individual.

9. In vitro method according to anyone of claims 1 to 6, which includes administering Klotho to the individual if the determined Klotho level is below a value corresponding to 100 pg/ml as determined in serum and preferably below a value corresponding to 200 pg/ml as determined in serum.

10. In vitro method according to claim 9, wherein administering Klotho includes administering a therapeutically effective number of mesenchymal stem cells which produce Klotho, or a therapeutically effective number of cells containing a nucleic acid vector which comprises a region encoding a Klotho protein, said region being operably linked to a promoter or promoter/enhancer combination, and said vector containing cells expressing Klotho.

11. In vitro method according to anyone of claims 1 to 6, which includes subjecting the individual to increased physical activity, especially physical training like muscle training and/or endurance training if the determined Klotho level is below a value corresponding to 100 pg/ml as determined in serum and preferably below a value corresponding to 200 pg/ml as determined in serum.

12. In vitro method according to anyone of claims 1 to 11, wherein the body fluid is saliva.

13. In vitro method according to anyone of claims 1 to 12, wherein the Klotho content is determined via immune assay including at least one antibody or aptamer which specifically binds Klotho, preferably wherein the immune assay is a double antibody sandwich assay or as a competitive assay, preferably performed as a lateral flow assay.

14. Test kit for performing the method according to anyone of claims 1 to 13, **characterized in that** it is an immunoassay test kit, preferably wherein the test is performed as a double antibody sandwich assay or a competitive assay.

15. Test kit according to anyone of claims 14 to 16, **characterized in that** is suitable for a lateral flow assay and contains a solid phase comprising:
a) a sample pad for application of a sample to be tested,
b) a conjugate or reagent pad comprising a conjugate of a Klotho specific antibody, which conjugate comprises a detectable label, or a conjugate of such antibody/label conjugate and Klotho,
c) a reaction or capture zone on which an antibody specific to the Klotho protein is immobilized, preferably provided in a line across the width of the solid phase, and
d) a wick or waste reservoir for collecting sample fluid and sample components and surplus reagent or conjugate,
and optionally further,
e) a control zone comprising an antibody specifically binding to the conjugate of step b), however, not to Klotho.
